# EUROPÄISCHE PATENTSCHRIFT

(11) **EP 4 578 430 B1**
(45) Veröffentlichungstag und Bekanntmachung des Hinweises auf die Patenterteilung: **19.08.2026**
(21) Anmeldenummer: 23220301.8
(22) Anmeldetag: 27.12.2023
(51) Int. Cl.: A61F 13/00, A61F 13/0206

(54) **DRUCKSTABILES, ZUSCHNEIDBARES WUNDVERSORGUNGSPRODUKT FÜR NEKROTISCHE ULCERA**
PRESSURE-STABLE, TRIMABLE WOUND CARE PRODUCT FOR NECROTIC ULCERS
PRODUIT D'ENTRETIEN DE PLAIE, STABLE À LA PRESSION, POUVANT ÊTRE COUPÉ POUR ULCÉREUX NÉCROSIQUES

(43) Veröffentlichungstag der Anmeldung: 02.07.2025
(73) Patentinhaber: Paul Hartmann AG, 89522 Heidenheim (DE)
(72) Erfinder: MARZ, Jacqueline, 8212 Neuhausen (CH); ROTHMAIER, Markus, 8212 Neuhausen (CH)
(74) Vertreter: Paul Hartmann AG Patents & Licensing

(56) Entgegenhaltungen:
- EP-B1- 3 315 145
- AU-A1- 2012 236 039
- DE-A1- 102013 105 063
- DE-U1- 202013 104 893

## Beschreibung

### Technisches Gebiet der Erfindung

Die Erfindung betrifft ein textiles, mehrschichtiges Wundversorgungsprodukt, welches geeignet ist zur Behandlung unterschiedlicher Wunden, insbesondere Ulcera und besonders Ulcera an der Fußsohle. Das Wundversorgungsprodukt ist durch seinen speziellen Aufbau in der Lage ein physikalisch-autolytisches Debridement durchzuführen, dadurch Nekrosen aus der Wunde schonend zu entfernen und hierdurch den Wundverschluss einzuleiten.

### Hintergrund der Erfindung

Wundauflagen für die feuchte Wundversorgung sind aus dem Stand der Technik grundsätzlich bekannt.

Eine Variante wird in der WO 2011/141454 A1 beschrieben. Es handelt sich hierbei um eine wundkissenartige oder kompressenartige Wundauflage, die auf eine Wunde aufbringbar ist oder auch zum Austamponieren tiefer Wunden verwendet werden kann. Bestandteil dieses Produkts ist ein Saug-/Spülkörper, welcher über eine umlaufende Schweißnaht verfügt und herstellerseitig mit einer salzhaltigen wässrigen Lösung beaufschlagt ist, wodurch das enthaltene superabsorbierende Material aufquillt und in einen gelartigen Zustand übergeht. Dies verleiht dem Saug-/Spülkörper eine duale Funktion bei Wunden mit starker Exsudation: Wundsekrete werden einschließlich der darin enthaltenen Bestandteile wie Keime durch den Saug-/Spülkörper aktiv aufgenommen und darin gehalten, wobei der Saug-/Spülkörper im Austausch die salzhaltige wässrige Lösung an die Wunde abgibt und somit ein feuchtes Wundmilieu schafft oder unterstützt. Hierdurch wird die Wundreinigung und eine positive Wundkonditionierung unterstützt und somit die Heilung positiv beeinflusst. Dies wird als interaktive Nasstherapie bezeichnet, die insbesondere bei schlecht heilenden Wunden, bei klinisch manifest infizierten Wunden oder bei chronischen Wunden mit unterschiedlicher Genese, wie diabetisches Gangrän, Dekubitalulcus oder Ulcus cruris bevorzugt Anwendung findet. Wundauflagen dieses Typs haben jedoch den Nachteil, dass sie (nach Herstellung) nicht dafür vorgesehen sind, an die Größe der Wunde angepasst, z.B. zugeschnitten, zu werden. Grund hierfür ist, dass beim Zuschneiden die äußere Barriere inklusive der Schweißnaht durchtrennt wird und das Gel anschließend herausquillt. Ein Zuschneiden im trockenen Zustand mit späterer Flüssigkeitszugabe ist ebenfalls nicht möglich, da nach dem Schneiden die Superabsorberpartikel aus dem Inneren herausfallen und schlimmstenfalls in der Wunde verbleiben. Auch ohne ein Zuschneiden des Produktes kann es passieren, dass unter starkem Druck die Schweißnaht reißt und das Gel bzw. die Partikel aus superabsorbierendem Polymer (SAP) austreten. Deswegen ist ein solches Produkt aus dem Stand der Technik nicht für die Behandlung von Ulcera an der Fußsohle, wie sie häufig bei Diabetikern auftreten, geeignet bzw. kann nur genutzt werden, wenn der Patient das Auftreten vermeidet, beispielsweise durch eine liegende Position oder eine Hochlagerung des betroffenen Beines. Andernfalls droht die Wundauflage zu platzen, sobald der Patient sein Gewicht auf den zu behandelnden Fuß verlagert. Die Behandlung im Liegen oder der Einsatz von Krücken sind belastend für den Patienten. Ausdauerndes Liegen erhöht das Thromboserisiko und Krücken empfinden vor allem ältere Patienten als übermäßig belastend.

Das in Bezug auf die WO 2011/141454 A1 Dargestellte gilt in nahezu identischer Weise für die Wundauflage der WO 2016/156619 A1. Auch diese ist für die feuchte Wundversorgung vorgesehen und wird in Form eines Flächenmaterials bereitgestellt, das aus mehreren Schichten besteht, die lediglich am Rand durch eine Schweißnaht miteinander verbunden sind. Die Schichten können sich ansonsten z.B. bei Körperbewegungen gegeneinander verschieben, so dass eine flexible Anordnung entsteht. Auch diese Wundauflage ist nicht zuschneidbar und nicht für die Anwendung an der Fußsohle geeignet.

Zwar können die oben genannten Produkte für die Versorgung von Ulcera, z.B. in Form eines offenen Beines, genutzt werden, allerdings entsteht auch hier häufig ein Problem: Da die Produkte nicht an die Größe der Wunde zugeschnitten werden können, überragen sie die Wunde zwangsläufig und da sie in feuchter Form appliziert werden, wird die gesunde Haut, welche die Wunde umgibt, mit zunehmender Anwendungsdauer aufquellen. In der Fachsprache wird dieser unerwünschte Effekt als Mazeration bezeichnet und kann im ungünstigsten Fall zu einer Vergrößerung der Wunde führen.

Weiterer Stand der Technik ist aus DE202013104893U1, DE102013105063, AU2012236039 und EP3315145 bekannt.

Folglich besteht ein Bedarf für eine Wundauflage, die für die Behandlung unterschiedlicher Wunden, insbesondere von Ulcera, und zwar auch an der Fußsohle, geeignet ist, zugeschnitten werden kann, Nekrosen entfernt und ein feuchtes Wundmilieu schafft, so dass der Wundverschluss gefördert wird, und zwar ohne, dass der Patient während der Behandlung in seiner Bewegungsfreiheit eingeschränkt wird. Ferner soll eine Mazeration der Wundränder vermieden werden.

### Zusammenfassung der Erfindung

Die vorangestellte Aufgabe wird gelöst durch die Bereitstellung eines

zuschneidbaren Wundversorgungsproduktes umfassend die folgenden Bestandteile
a) eine erste flüssigkeitsdurchlässige Schicht mit einem Vliesstoff,
b) eine zweite flüssigkeitsdurchlässige Schicht mit einem Vliesstoff,
c) eine zwischen der ersten Schicht und der zweiten Schicht gelegene Quellschicht in Form eines Vliesstoffes mit superabsorbierenden, polymerhaltigen Fasern, wobei die superabsorbierenden Fasern mit mindestens einer anderen Faserart in einem Filz vorliegen,
d) eine wässrige Salzlösung, die in der Quellschicht eingelagert ist, im eingelagerten Zustand einen pH < 7,0 hat und die während einer Wundbehandlung an eine Wunde abgegeben werden kann,
wobei die erste Schicht, die Quellschicht und die zweite Schicht einen identischen Zuschnitt haben und ohne Überstand vollflächig aneinanderliegen, und wobei das Wundversorgungsprodukt nahtlos ausgestaltet ist.

Alternativ kann das Wundversorgungsprodukt aus den oben genannten Bestandteilen bestehen.

Durch den beschriebenen Aufbau kann die erfindungsgemäße Wundauflage auf die Größe der Wunde zugeschnitten werden und falls eine Anwendung an der Fußsohle vorgesehen ist, bleibt der Patient während der Behandlung mobil. Weiterhin wird ein mögliches Überstehen der Wundauflage über die Fußsohle hinaus vermieden, so dass z.B. das Tragen von Schuhen weiterhin möglich ist. Bei einer Verlagerung des Körpergewichts auf die Wundauflage wird die Wunde verstärkt über die Salzlösung gespült und bei der nachfolgenden Entlastung (Belastung des anderen Beins) wird die Lösung größtenteils wieder von der Wundauflage aufgenommen. Hierdurch erfolgt ein repetitives physikalisch-autolytisches Debridement, welches Zelltrümmer, Eiter und vor allem nekrotische Beläge entfernen kann. Gleichzeitig werden - im Falle einer Wundinfektion - Erreger aus der Wunde gespült und von der Wundauflage aufgenommen, wo sie zurückgehalten werden. Zusammen mit dem feuchten Wundmilieu werden optimale Bedingungen geschaffen, um insbesondere auch chronische Wunden zum Abheilen anzuregen und die Lebensqualität der betroffenen Patienten zu steigern.

### Detaillierte Beschreibung der Erfindung

Der Ausdruck "zuschneidbar" meint, dass ein Produkt oder Material mit einem handelsüblichen mechanischen Werkzeug wie beispielsweise einer Schere gezielt vor der Anwendung auf eine vom Anwender vorgesehene Größe verkleinert werden kann, ohne dass das Produkt oder Material an Funktion einbüßt, und ohne dass ein unerwünschter (teilweiser oder vollständiger) Zerfall des Produktes oder Materials einsetzt.

Der Begriff "medizinisch akzeptables Material" im Sinne der Erfindung ist eine ungiftige, fusselfreie und beständige Substanz, die sich unter Normalbedingungen weder in polaren noch in unpolaren Substanzen zersetzt und weder von den Absonderungen tierischer noch bakterieller Zellen in nennenswertem Ausmaß abgebaut werden kann.

Der Ausdruck "atraumatisch" meint, dass ein Wundversorgungsprodukt sich nicht mit der Wunde fest verbindet, also beispielsweise nicht in der Wunde eintrocknet oder mit dieser verwächst und dass eine schmerzfreie Entfernung des Produktes ohne Störung des Heilungsprozesses möglich ist.

Unter dem Ausdruck "konfiguriert zum Auflegen auf eine Wunde" wird verstanden, dass das Wundversorgungsprodukt zum Auflegen auf eine Wunde zu einem therapeutischen Zweck im Sinne einer Wundbehandlung vorgesehen ist.

Unter "Ringerlösung" wird eine wässrige Lösung mit Natriumchlorid, Kaliumchlorid und Calciumchlorid (insbesondere 8,6 g NaCl, 0,3 g KCI und 0,33 g CaCl2 je Liter Wasser) verstanden, welche im Wesentlichen isotonisch ist (Osmolarität von etwa 308 mOsm/l). Ringerlösung wird in der Regel vor dem Einsatz sterilisiert.

"Proximal" meint im Sinne der vorliegenden Erfindung, dass ein Material oder ein Stoff eine Position innerhalb eines Wundversorgungsproduktes einnimmt, so dass er innerhalb dieses Produktes während der Anwendung in der Wundversorgung zur Wunde hin angeordnet ist. Dies ist beispielsweise bei einer Wundkontaktschicht der Fall.

"Distal" meint im Sinne der vorliegenden Erfindung, dass ein Material oder ein Stoff eine Position innerhalb eines Wundversorgungsproduktes einnimmt, so dass er innerhalb dieses Produktes während der Anwendung in der Wundversorgung von der Wunde weg angeordnet ist, wie es beispielsweise bei einem Backing (abschließende Stützschicht) der Fall ist.

"SAF" ist eine Abkürzung, die für superabsorbierende Fasern steht. Derartige Fasern sind in der Lage ein Vielfaches ihres Eigengewichts an polaren Flüssigkeiten zu absorbieren und können zusammen mit anderen Fasern zu einem stabilen Faserverbund verarbeitet werden.

"Zusatzfaser" meint eine Faser, die sich von superabsorbierenden Fasern (SAF) unterscheidet und somit selbst keine SAF ist.

"Moisture Vapor Transmission Rate" oder "MVTR" meint einen Messwert, der die Durchlässigkeit von Wasserdampf durch ein bestimmtes Material in g/m2/24h, angibt. Die MVTR kann mithilfe des Standards DIN EN 13726-2 (Juni 2002) bestimmt werden.

Das erfindungsgemäße Wundversorgungsprodukt besteht aus mindestens drei Schichten: erste Schicht, Quellschicht, zweite Schicht. Alle drei dieser Schichten haben einen identischen Zuschnitt, so dass sie ohne Überstand vollflächig aneinanderliegen. Geringe produktionsbedingte Abweichungen (Toleranzen) sind jedoch möglich, ohne dass die Funktion eingeschränkt wird. Dabei können eine oder zwei Schichten bis zu 5 mm über eine andere Schicht bzw. Schichten hinausragen.

Die Druckresistenz wird vermittelt durch den Aufbau des Produktes, wobei die Zusammensetzung der Quellschicht umfassend einen Filz aus SAF und Zusatzfasern sowie der dadurch ermöglichte Verzicht auf eine Schweißnaht entscheidend sind. Nahtlos bedeutet insbesondere, dass das Produkt über keine Schweißnaht verfügt. Auf diese Wiese können die erforderlichen Druckbelastbarkeitswerte erreicht werden. Demgemäß reagiert das Wundversorgungsprodukt unter Einwirkung eines mechanischen Drucks lediglich durch elastische und somit reversible Verformung. So kann bevorzugt vorgesehen sein, dass das Wundversorgungsprodukt eine Druckresistenz gegenüber einem Druck von mindestens 8 kg / 100 cm2 , bevorzugt 20 kg / 100 cm2, noch besser mindestens 50 kg / 100 cm2 und am besten 120 kg / 100 cm2 aufweist.

Die Druckresistenz kann mithilfe des folgenden Verfahrens getestet werden:
1) Bereitstellen eines mit wässriger Salzlösung behandelten Wundversorgungsproduktes, dessen Sterilisation innerhalb der letzten 14 Tagen stattgefunden hat.
2) Einlegen des (unverpackten) Produktes in einen handelsüblichen, transparenten ZIP-Beutel
3) Aufbau der zu testenden Gewichtskraft innerhalb von einer Sekunde
4) Halten der Gewichtskraft für eine Sekunde
5) Abbau der Gewichtskraft innerhalb von zwei Sekunden
6) Wiederholung der Schritte 3 bis 5, so dass die Gewichtskraft insgesamt zehn Mal appliziert wird
7) Entnahme des Produktes aus dem Beutel
8) Visuelle Inspektion sowohl des Produktes als auch des Beutelinneren auf abgetrennte Fasern oder ausgetretenes Gel

Wenn weder am Produkt selbst noch im Beutelinneren abgetrennte Fasern oder ausgetretenes Gel erkennbar sind, gilt der Test für die entsprechende Gewichtskraft als bestanden.

Bevorzugt ist das Wundversorgungsprodukt derlei gestaltet, dass es aufgrund seiner elastischen Eigenschaften und der Druckbelastbarkeit um 90°, noch besser um 180° entlang einer tatsächlichen oder (bei unsymmetrischem Aufbau) gedachten Mittellinie umgeschlagen bzw. umgefaltet werden kann, und zwar ohne, dass eine plastische Verformung stattfindet.

Erste und zweite Schicht können neben dem genannten Vliesstoff auch andere Bestandteile wie z.B. andere Faseranordnungen umfassen, solange diese nach einem Zuschneidevorgang nicht desintegrieren. Bevorzugt bestehen erste und zweite Schicht vollständig aus Vliesstoff, da Vliesstoffe besonders resistent gegen Zerfaserungen sind, und zwar auch nach einem Zuschneiden des Materials.

Die Quellschicht enthält superabsorbierende Fasern (SAF), insbesondere superabsorbierende Fasern, welche ein superabsorbierendes Polymer enthalten. Das superabsorbierendes Polymer umfasst bevorzugt ein acrylathaltiges Polymer oder ein Acrylat-Co-Polymer. Die SAF können einen Faserdurchmesser von beispielsweise 50 bis 500 µm haben. Bevorzugt wird ein Faserdurchmesser von 100 bis 200 µm, da sich bei diesem Durchmesser ein ideales Wasser-zu-Faser-Verhältnis ausbilden kann. Hierdurch kann eine größere Menge, beispielsweise 15 ml, einer wässrigen Salzlösung aus der Quellschicht an die Wunde abgegeben, aber auch wieder aufgenommen werden. Dieser Mechanismus basiert zum einen auf chemisch-physikalischen Prozessen wie der Diffusion, bei welcher saubere Salzlösung in Richtung Wunde wandert und ausgespültes Wundexsudat in Richtung Quellschicht wandert. Zum anderen wird die Salzlösung unter mechanischem Druck teilweise herausgepresst und beim Aufheben der Druckkraft wieder von der Quellschicht aufgenommen. Möglichkeiten die SAF bereitzustellen und anzupassen werden an anderer Stelle näher erläutert.

Die Erfindung umfasst ein zuschneidbares Wundversorgungsprodukt, wobei hierunter ein Schneiden verstanden wird, bei welchem alle Schichten des Produktes zeitgleich durchtrennt werden. In der Regel wird das Schneiden dazu führen, dass ein Bereich vom Gesamtprodukt abgetrennt und die Fläche des Produktes somit verkleinert wird. Es ist allerdings auch möglich das Produkt lediglich einzuschneiden, um es entlang der Einschnittkante zu biegen oder zu falten. Dies kann in Einzelfällen bei der Wundversorgung von Fingern oder Zehen vorteilhaft sein, da sich das Produkt auf diese Weise besonders gut an die Körperkonturen anpassen lässt. Falls das Wundversorgungsprodukt als Tamponade verwendet wird, kann diese durch den Schneidevorgang auch verkürzt werden, um an die Tiefe der zu vorsorgenden Kavität angepasst zu werden, was einen großen Vorteil gegenüber den bislang im Stand der Technik verwendeten Produkten zur Tamponierung darstellt: Die bislang üblichen Artikel sind entweder nicht zu schneidbar oder falls sie zuschneidbar sind, besteht das Risiko des Einwachsens von organischem Gewebe in die Produktstruktur. Letzterem wird in der Praxis häufig versucht zu begegnen, indem die benötigte Menge eines Standard-Verbandsmaterials (wie z.B. Gaze) mit hydrophober Salbe durchtränkt und anschließend in die Kavität eingebracht wird. In Anbetracht der bei Kavitäten häufig täglich notwendigen Verbandswechsel, ist der mit diesem Ansatz einhergehende Arbeitsaufwand für das medizinische Personal enorm.

Bevorzugt ist das Wundversorgungsprodukt flächig, hat also eine flache distal gelegene Oberseite und eine ebenfalls flache proximal gelegene Unterseite, so dass es hervorragend zur Wundabdeckung geeignet ist und bündig mit den Wundrändern abschließt. Flach meint, dass die jeweilige Schicht keine wesentlichen Erhebungen oder Vertiefungen aufweist. Sowohl die erste als auch die zweite Schicht können als Unterseite bzw. als Wundkontaktschicht fungieren. Hierdurch lässt sich das Produkt gut auf oder in einer Wunde drapieren. Die flache Unterseite unterstützt die atraumatischen Eigenschaften und vermindert die Wundreizung.

Das Wundversorgungsprodukt ist darüber hinaus bevorzugt rund oder ellipsenförmig (in der Aufsicht). Dies hat im Vergleich zu rechteckigen Formen den Vorteil, dass das Fabrikat bereits im ungeschnittenen Zustand der Form der meisten chronischen Wunden ähnelt, welche (im Gegensatz zu z.B. Schnittwunden) in dem meisten Fällen eine kreisähnliche Erscheinung haben. Zudem hat sich gezeigt, dass runde oder ellipsenförmige Formen besser und dauerhafter an der Fußsohle fixiert werden können und den Träger weniger stören als andere Formen wie z.B. rechteckige Ausgestaltungen mit abgerundeten Ecken, wie sie häufig bei Schürfwunden zum Einsatz kommen. Zudem haben rechteckige Ausgestaltungen den Nachteil, dass sie sich bei fortwährender mechanischer Beanspruchung - wie z.B. beim Gehen - im Bereich der Ecken schnell von der Haut abzulösen beginnen. Dabei zeigen die Ecken eine Tendenz sich aufzurollen, was zu einer ungewollten Erhebung führt und im Bereich der Fußsohle zu Druckschmerzen führen kann.

Bei tiefen Gewebedefekten - sogenannten Kavitäten - kann das erfindungsgemäße Wundversorgungsprodukt in die Wunde eintamponiert werden. Dies hat neben den bereits erwähnten Vorteilen der Wundversorgung zusätzlich den Effekt, dass die Kavität sich nicht vorzeitig auf Höhe der Epidermis verschließt, sondern dass das Nachwachsen von Gewebe von zentral (aus dem Boden des Wundbetts) nach peripher (zur Hautoberfläche) geschieht. Andernfalls könnte der vorzeitige Wundverschluss zu einem Abszess führen und die weitere Versorgung erheblich erschweren.

Das erfindungsgemäße Wundversorgungsprodukt enthält eine Quellschicht in Form eines Vliesstoffes mit superabsorbierenden, polymerhaltigen Fasern, wobei die superabsorbierenden Fasern mit mindestens einer anderen Faserart (Zusatzfaser) in einem Filz als Faserverbund vorliegen. Sinn und Zweck dieser weiteren Fasern ist es der Quellschicht ausreichende Reiß- und Zugfestigkeit sowie Formstabilität im feuchten Zustand zu verleihen und eine (außenliegende) Naht zu vermeiden. Da die SAF mit der Zusatzfaser als Filz vorliegen, bilden diese auch nach einem Schneidevorgang einen stabilen Faserverbund. Durch den Verzicht auf eine Naht steigt die Druckresistenz erheblich an.

In Bezug auf die Quellschicht kann es sich bei der Zusatzfaser ("anderen Faser") um eine Faserart synthetischen, natürlichen oder halb-synthetischen Ursprungs handeln, wobei SAF explizit ausgenommen sind. Beispiele für geeignete Fasern synthetischen Ursprungs sind Polyolefin-basierte Fasern wie Polyester und Polyamid. Innerhalb der Gruppe der Polyester ist Polyethylenterephthalat besonders geeignet. Beispiele für geeignete Fasern natürlichen Ursprungs sind wie Baumwolle und Flachs. Ein Beispiel für Fasern halb-synthetischen Ursprungs ist Lyocell. Natürliche und halb-synthetische Fasern bilden gemeinsam die Gruppe der zellulosehaltigen Fasern, sofern sie Zelluloseanteile enthalten.

Die Zusatzfaser liegt zusammen mit der SAF in einem Filz, bevorzugt einem Nadelfilz, vor. Das heißt, dass die beiden Fasertypen gemeinsam verfilzt sind. Der Filz kann als Nassfilz oder als Trockenfilz hergestellt werden. Die Herstellung als Trockenfilz ist bevorzugt, da so eine Rücktrocknung der SAF vermieden werden kann. Der Trockenfilz kann als Nadelfilz, Klebefilz oder thermisch verfestigtes Schmelzfilz bereitgestellt werden. Bei einem Nadelfilz werden die Zusatzfasern und die SAF maschinell miteinander zu einem Filz vernadelt. Bei einem Klebefilz werden die beiden Faserarten durch Beimengen eines Klebstoffs (hauptsächlich durch chemische Wechselwirkung) miteinander verbunden. Der verwendete Klebstoff sollte nach der Aushärtung nicht wasserlöslich sein, da es ansonsten durch Einwirkung der wässrigen Salzlösung zu einem Zerfall kommen könnte. Bei einem Schmelzfilz wird die Zusatzfaser aus dem Bereich der Schmelzfasern gewählt und mit den SAF (welche inhärente Schmelzfasereigenschaften besitzen) durch Einsatz thermischer Energie miteinander verbunden. Bevorzugt liegen die Fasern der Quellschicht als Nadelfilz vor. Eine Quellschicht in Form eines Nadelfilzes bietet den Vorteil, dass sie kompatibel zu sämtlichen Sterilisationsmethoden ist, da sie hervorragende Beständigkeit gegenüber Hitze, Druck, Wasserdampf, Strahlung und Ethylenoxid zeigt. Darüber hinaus lässt die Nadelfilzform große Freiheit bei der Auswahl der Fasern und weist eine sehr lange Haltbarkeitsdauer selbst bei langer Lagerung auf.

Die derart aufgebaute Quellschicht kann problemlos geschnitten werden, ohne dass sich die SAF und die Zusatzfaser voneinander lösen und, ohne dass Superabsorberpartikel, wie sie häufig in absorbierenden Wundauflagen verwendet werden, aus der Quellschicht im Schnittbereich herausfallen. Des Weiteren wird eine dauerhafte Befestigung an den anderen Schichten des Wundversorgungsproduktes ermöglicht. So ist es beispielsweise möglich die Quellschicht auf ihrer distalen und proximalen Seite mit dem Vliesstoff der ersten und zweiten Schicht zu verkleben und zwar auch, wenn diese andere Materialien als die Quellschicht enthalten. Eine Möglichkeit, wie ein derartiges Laminat erhältlich ist, wird in Ausführungsbeispiel 1 beschrieben.

Das erfindungsgemäße Wundversorgungsprodukt ist hervorragend geeignet für die feuchte Wundbehandlung. Dabei meint "geeignet für die feuchte Wundbehandlung" bzw. "zur feuchten Wundbehandlung", dass das Produkt ohne weitere Vorbereitung oder Modifikation derart eingesetzt werden kann, dass die feuchte Wundbehandlung unmittelbar mit der Applikation des Produktes einsetzt. Damit einhergehend kann das Wundversorgungsprodukt zwischen Herstellung und Einsatz im feuchten Zustand gelagert werden, ohne dass es zu einer Beeinträchtigung der strukturellen Integrität kommt.

Die in der Quellschicht enthaltene Salzlösung spült die Wunde, löst hierdurch Belege wie z.B. Fibrin, unterstützt die autokatalytische Zersetzung von Nekrosen und entfernt Mikroorganismen. Überschüssige Matrix Metalloproteinasen werden aus der Wunde gespült und stagnierte Heilungsprozesse neu angestoßen. Das feuchte Milieu beschleunigt zudem generell den Wundverschluss.

Sofern das Produkt an der Fußsohle angebracht ist, wird durch die Bewegung der venöse Abfluss des Blutes angeregt, was vor allem bei chronischen Wunden, die auf Venenstau zurückzuführen sind (z.B. Ulcus cruris auch bekannt als "offenes Bein") von großer Bedeutung ist. Da die Form des Produktes durch das Zuschneiden an die Form der Wunde angepasst werden kann, wird ein Überragen der Wunde und damit eine Mazeration der die Wunde umgebenden Haut trotz der feuchten Wundversorgung vermieden. Zudem lässt sich das Produkt auf diese Weise deutlich komfortabler unter eine Kompressionsbandage oder einem Kompressionsstrumpf tragen, wie sie bei Druckgeschwüren zum Einsatz kommen, um die pathologische venöse Stauung zu beheben.

Das Wundversorgungsprodukt gemäß der vorliegenden Erfindung ist hervorragend zur Behandlung, insbesondere zur feuchten Wundbehandlung, von schwer oder nicht heilenden und chronischen Wunden geeignet. Hierunter fallen insbesondere die folgenden Wundarten: diabetische Fußläsionen, Ulcus cruris venosum, Ulcus cruris arteriosum, Ulcus cruris mixtum und Dekubitus. Diese Wunden zeichnen sich häufig durch die Anwesenheit von Belägen (z.B. Fibrinbelägen) und Nekrosen aus. Weiterhin scheint der gehemmte Heilungsprozess mit einem Ungleichgewicht der notwendigen Botenstoffe, Enzyme (z.B. Matrix-Metalloproteasen) und molekularen Inhibitoren in der Wunde einherzugehen. Durch die Spülung der Wunde im Rahmen der feuchten Wundbehandlung wird das zuvor vorherrschende chemische Ungleichgewicht beseitigt und es kann sich ein für die Wundheilung förderliches Milieu einstellen. Die zuvor gehemmte Heilung wird auf diese Weise wieder in Gang gesetzt. Wie bereits erwähnt, lässt sich das erfindungsgemäße Wundversorgungsprodukt in den allermeisten Fällen mit einer Kompressionstherapie kombinieren, um den venösen Rückstau als ursächliches Ulcus-auslösendes Problem, zu beseitigen.

Besonders geeignete Mittel, um das erfindungsgemäße Wundversorgungsprodukt an der Wundstelle oder in einer tiefen Wunde (Kavität) zu fixieren, sind Sekundärverbände wie z.B. Klebefolien, Gaze, Bandagen und Rollenpflaster. Unter diesen Mitteln sind Klebefolien bevorzugt, da sie - wie das Wundversorgungsprodukt selbst auch - zugeschnitten werden können, ohne an Funktion einzubüßen oder auszufransen. Weiterhin reduzieren Klebefolien die Verdunstung und behindern auch beim Anbringen an der Fußsohle den Patienten beim Gehen nicht. Darüber hinaus können Klebefolien wasserdicht ausgestaltet werden und dadurch das Wundversorgungsprodukt von unerwünschten Außeneinwirkungen (z.B. beim Duschen) schützen.

Der Vliesstoff der ersten Schicht und / oder der zweiten Schicht kann thermisch verfestigt sein und somit thermisch verfestigte Fasern enthalten. Auf diese Weise wird die Bindungsfestigkeit erhöht und gleichzeitig können leichtere Vliesstoffe generiert werden, was zu einer Materialeinsparung führt. Gleichzeitig bleibt die Wasserdurchlässigkeit erhalten. Der thermisch verfestigte Vliesstoff kann thermoplastische Fasern enthalten. Beispiele hierfür sind Polypropylen (PP), Polyvinylchlorid (PVC), Polyethylen (PE), Polyester (PET), Polyacryl (PC), Polyacrylnitril (PAN), Polyamid (PA), Polyurethan (PU), Viskose (CV) und Mischungen aus zwei oder mehr dieser Fasertypen. Ferner können thermoplastische Zusatzkomponenten enthalten sein. Ein Beispiel für eine thermoplastische Zusatzkomponente ist PET.

Zur Herstellung eines geeigneten thermisch verfestigten Vliesstoffes können die zu einem lockeren
Vlies gelegten Fasern bis zum Schmelzpunkt erwärmt werden, wodurch sie sich verbinden. Zum Einsatz kommen kann die Kalanderverfestigung (auch Thermobonding genannt) oder die Heißluftverfestigung (auch Thermofusion genannt), wobei im Falle von PP-Fasern sich besonders die Kalanderverfestigung bewährt hat.

Bei Einsatz von Fasern, die sich nicht in Reinform für die thermische Verfestigung eignen, können Bindefasern beigemengt werden, um auf diese Weise dennoch eine thermische Verfestigung zu ermöglichen. So kann das thermisch verfestigte Vlies neben thermoplastischen Fasern und thermoplastischen Zusatzkomponenten auch andere Faserarten wie z.B. Baumwolle oder Viskose enthalten. Bevorzugt handelt es sich bei diesen Fasern um Fasern natürlichen Ursprungs oder teilsynthetische Fasern wie Viskose.

Die Wärmeübertragung für die thermische Verfestigung kann mittels Wärmeleitung, Konvektion oder Strahlung erfolgen. Besonders geeignet sind das Kalander- und das Heißluftverfahren. Diese Art der Verfestigung ermöglicht die Umwandlung eines losen Faservlieses in einen festen, beständigen Vliesstoff. Das Risiko, das Fasern sich lösen und in die Wunde gelangen ist auf diese Weise stark reduziert.

Die SAF in der Quellschicht können ein Polymer umfassen oder aus einem Polymer bestehen, das Acrylat enthält. Es kann sich hierbei um ein Polyacrylamid oder ein Derivat eines Polyacrylamids oder um ein Acrylamid-Copolymer handeln. Gemäß einer bevorzugten Ausführungsform der Erfindung enthalten die SAF in der Quellschicht des Wundversorgungsprodukts ein Polymer, welches quervernetzbar ist. Ein Beispiel für quervernetzbare Polymere, aus denen SAF gewonnen werden können oder die einen Bestandteil von SAF bilden können, sind acrylahaltige Polymere.

Die SAF in der Quellschicht des Wundversorgungsprodukts können vorzugsweise ein quervernetztes Polymer enthalten. Bevorzugt beruht die Quervernetzung auf der Bildung von Ester-Verbindungen. Das quervernetzte Polymer kann durch diese Ester-Bindungen quervernetzt oder quervernetzbar sein. Durch den Grad der Quervernetzung kann die Biegsamkeit bzw. der Widerstand der SAF (Biegewiderstandsmoment) in Anwesenheit der wässrigen Salzlösung eingestellt werden. Je höher der Quervernetzungsgrad, desto zäher wird die gelartige Konsistenz der SAF, die in Anwesenheit von Wasser oder anderen wässrigen Flüssigkeiten wie beispielsweise Wundexsudat als Hydrogele vorliegen können. Gleichzeitig steigt mit dem Quervernetzungsgrad die Belastbarkeit der Quellschicht und damit des Wundversorgungsproduktes insgesamt.

Die Quervernetzungsbindungen können Acrylesterbindungen sein. Die Vernetzung kann über radikalische oder kationische Polymerisationsmechanismen oder andere Ver- oder Umesterungsmechanismen wie beispielsweise die Michael-Addition, erfolgen. Bevorzugt wird die radikalische Polymerisation. Polyacrylamide können quervernetzt werden, indem sie in ein saures Milieu eingebracht werden. Je nachdem mit welcher Geschwindigkeit die Reaktion erfolgen soll, kann der pH-Wert hierzu kleiner als 6,0, kleiner als 5,0 oder kleiner als 4,0 sein. Beispielsweise kann der pH-Wert im Bereich von 3,0 bis 6,0 oder 1,0 bis 5,0 oder 0,0 bis 4,0 liegen.

In diesem Zusammenhang können die SAF ein Polymer enthalten, dass zu 5 bis 50 % quervernetzt ist, bevorzugt 10 bis 40 %. Bei acrylhaltigen Polymeren kann der Quervernetzungsgrad anhand der Menge der über Ester-Bindungen quervernetzten Acrylgruppen innerhalb des Polymers bestimmt werden. Bei einem Quervernetzungsgrad von 50 % wäre somit die Hälfte der Acrylgruppen quervernetzt.

Ferner können die SAF ein Co-Polymer enthalten, bei dem es sich um ein Acryl-Co-Polymer handeln kann.

Ein mögliches Beispiel ist Poly(Acrylsäure-Co-Acrylamide)/Polyvinylalkohol. Dieser kann direkt zu SAF gesponnen werden.

Die Quervernetzung der SAF steht deren Vernadelung mit mindestens einer anderen Faserart (einer Zusatzfaser, die keine SAF ist) zu einem Nadelfilz nicht entgegen. Die Quervernetzung kann sowohl vor der Vernadelung als auch im Anschluss stattfinden, wobei empfohlen wird, diese vor der Vernadelung vorzunehmen, da auf diese Weise der Quervernetzungsgrad genauer eingestellt werden kann. Eine thermische Verfestigung kann sich an die Vernadelung anschließen, wobei diese drei Maßnahmen (Quervernetzung, Vernadelung und thermische Verfestigung) sich gegenseitig synergistisch ergänzen und in einer Quellschicht resultieren, die hervorragende Feuchtigkeitsspeicherung, Feuchtigkeitsabgabe und ausgezeichnete mechanische Festigkeit selbst nach einem Zuschneiden durch den Endverbraucher oder Pflegekräfte aufweist.

Das Verhältnis zwischen SAF und Zusatzfaser in der Quellschicht kann beispielsweise folgendermaßen aussehen: 20 Gew.-% bis 80 Gew.-% SAF und 80 Gew.-% bis 20 Gew.-% Zusatzfasern. Bevorzugt beträgt der Anteil der SAF in der Quellschicht 20 Gew.-% bis 60 Gew.-%, besonders bevorzugt 20 Gew.-% bis 40 Gew.-%, wobei der übrige Anteil durch die Zusatzfasern gestellt wird. Eine mögliche Ausführungsform enthält 25 Gew.-% SAF und 75 Gew-% Polyolefin-basierte Fasern in der Quellschicht.

Erfindungsgemäß sind die Quellschicht, die erste Schicht und die zweite Schicht vollflächig (also über die gesamte Fläche der jeweiligen Ober- bzw. Unterseite) miteinander verbunden. Die Flächen können adhäsiv miteinander verbunden sein, wobei die adhäsive Verbindung der Flächen miteinander direkt (direkt angrenzend) oder indirekt - z.B. durch das Einfügen weiterer Zwischenschichten wie beispielsweise eine perforierte beidseitige klebende Folie oder eine poröse Schmelzfolie (thermoplastisch) - ausgestaltet sein kann. Die Schichten können auf adhäsive Weise beispielsweise durch einen Kleber bzw. eine Klebeverbindung zusammengefügt bzw. verbunden sein. Die Klebeverbindung bzw. Klebeschicht kann im fertigen Produkt als kohäsive Schicht vorliegen, das heißt innerhalb dieser ziehen die Teilchen des Klebers (Atome, Moleküle etc.) sich gegenseitig an. Bevorzugt handelt es sich um einen Schmelzkleber bzw. eine Schmelzkleberverbindung. Beispiele für mögliche Schmelzkleber sind Co-Polymere und Polyolefin-basierte Schmelzkleber wie Polyester bzw. Polyesterderivate wie Co-Polyester. Derartige Schmelzkleber sind hervorragend geeignet, um Vliesschichten, insbesondere solche welche synthetische Fasern enthalten, dauerhaft zusammenzufügen. Sie sind darüber hinaus mit einer späteren Hitze-Sterilisation in aller Regel technisch kompatibel. Der Schmelzpunkt kann dabei auch unter der späteren Sterilisationstemperatur liegen, da das Produkt während der Sterilisation sich bereits in einer Umverpackung befindet, die dem Fabrikat zusätzliche Stabilität verleiht. So kann der Schmelzpunkt des Schmelzklebers bzw. der Schmelzkleberverbindung beispielsweise bei 45 °C bis 120 °C, bevorzugt bei 45 °C bis 70 °C liegen. Bei Bedarf können - z.B. durch den Einsatz von Co-Polyester - Höchstwerte beim Schmelzpunkt von bis 140 °C und teilweise sogar darüber hinaus erreicht werden. Es wird empfohlen das Wundversorgungsprodukt im feuchten Zustand (also mit der enthaltenen wässrigen Salzlösung) zu sterilisieren, da auf diese Weise niedrige Sterilisationstemperaturen (z.B. 120 °C) bereits ausreichend sein können, sofern für eine ausreichende Einwirkungsdauer gesorgt wird.

Die oben genannten Schmelzkleber können dem Schmelzprozess in unterschiedlichen Ausgangsmaterialien zugeführt werden. So ist es möglich den Schmelzkleber in Form von Partikeln, als Netz oder als Folie bereitzustellen. Die Bereitstellung in Form von Partikeln oder als Netz wird bevorzugt, weil hierdurch die Beweglichkeit des resultierenden Produktes nicht eingeschränkt wird. Partikel bieten zudem den Vorteil der Rieselfähigkeit. Folien und Netze sind besonders geeignet, wenn unterschiedliche Schmelzkleber miteinander kombiniert werden sollen. Dabei können Schmelzkleberkombinationen zu besonders belastbaren Klebeverbindungen führen, wobei einer der verwendeten Kleber beim Herstellungsprozess die jeweiligen Schichten fixiert und der andere Kleber (mit höherem Schmelzpunkt) während des Sterilisationsprozesses reagiert und die Initialverbindung weiter verstärkt, ohne dass zusätzliche thermische Energie während des Prozesses aufgewandt werden müsste.

Die Schichten des Wundversorgungsproduktes - insbesondere die erste Schicht, die Quellschicht und die zweite Schicht - können als Laminat vorliegen. Dazu können diese entweder durch Einsatz von thermischer Energie miteinander verbunden werden und / oder mithilfe von Klebstoffen. Bei Einsatz von Klebstoffen, die nicht erhitzt werden, entsteht ebenfalls ein Laminat, welches in diesem Fall ein Kaltlaminat darstellt. Empfohlen wird zwischen den Schichten den bereits erwähnten Schmelzkleber aufzutragen, um das erhaltene Laminat beständiger zu machen. Die benötigten Temperaturen hängen von den zu behandelnden Materialien ab und können beispielsweise im Bereich von 45 - 160 °C liegen. Normalerweise genügt eine Einwirkungsdauer von 2 Sekunden bis 10 Minuten. Die optimale Einwirkungsdauer variiert in Abhängigkeit von der gewählten Temperatur, wird jedoch in den meisten Fällen im Bereich von 2 bis 30 Sekunden liegen.

Die auf diese Weise kohäsiv zusammengefügten Schichten des Wundversorgungsproduktes haben den Vorteil, dass es zur Ausbildung einer flächigen Verbindung kommt, die auch nach einem möglichen Zuschneiden des Produktes die Schichten weiterhin zusammenhält. Bevorzugt handelt es sich bei der flächigen Verbindung um eine vollflächige adhäsive Verbindung, besonders bevorzugt um eine vollflächige Klebeverbindung und im Optimalfall um eine vollflächige Schmelzkleberverbindung. Umgekehrt führen Methoden, welche die einzelnen Schichten lediglich entlang deren Rändern zusammenfügen (z.B. klassisches Schweißen) nicht zu einem zuschneidbaren Produkt.

Andere Möglichkeiten die Schichten miteinander zu verbinden, bestehen darin, Fasern der einen Schicht in einer angrenzenden Schicht zu verankern. Auf diese Weise können zwei Schichten beispielsweise durch Verweben oder Verfilzen miteinander verbunden sein.

Das Wundversorgungsprodukt lässt sich in zwei oder mehr Segmente zuschneiden. Alle auf diese Weise erhaltenen Segmente sind in der Wundversorgung einsetzbar. Die durch das Zuschneiden erhaltenen Segmente sind in der Lage, die in der Quellschicht eingelagerte wässrige Salzlösung an die Wunde abzugeben und diese dabei zu spülen und zu reinigen.

Ein weiterer Aspekt der Erfindung betrifft ein zuschneidbares flächiges Wundversorgungsprodukt, bei dem die erste Schicht, die Quellschicht und die zweite Schicht flächig über eine Schmelzverbindung zusammengefügt sind und wobei die Schmelzverbindung ein eingeschmolzenes Polyolefin oder eingeschmolzenes Copolymer enthält oder daraus besteht. Ein derartiges Laminat lässt sich vorteilhaft zuschneiden, ohne an den Schnittkanten auszufransen.

Ein besonderer Vorteil des erfindungsgemäßen zuschneidbaren flächigen Wundversorgungsproduktes ist, dass der vom Wundversorgungsprodukt ausgehende Spüleffekt bei wiederholter reversibler (nicht plastischer) Verformung oder bei wiederholter Druckkompression des Wundversorgungsproduktes verstärkt bzw. intensiviert wird. Dies kann in der Praxis erreicht werden durch z.B. Anbringung des Wundversorgungsproduktes an der Fußsohle und anschließender wiederholter Druckbelastung und Druckentlastung beim Gehen durch den Patienten bzw. Endanwender. Auf diese Weise wird beim Auftreten das Wundversorgungsprodukt durch das Körpergewicht komprimiert und der nicht fest an das SAF gebundene Anteil der wässrigen Salzlösung aus dem Wundersorgungsprodukt teilweise herausgedrückt. Sobald der Patient den Fuß hebt und das Wundversorgungsprodukt sich wieder ausdehnt, wird ein Anteil der abgegebenen Salzlösung (zusammen mit gelösten Stoffen, Erregern etc.) wieder aufgenommen. Dieser Vorgang findet während des Gehens in repetitiver Weise statt, was eine besonders intensive Spülung der Wunde ermöglicht.

Eine andere Möglichkeit, beispielsweise bei der Behandlung eines offenen Beines, ist das Anbringen des Wundversorgungsproduktes und anschließendes Umwickeln mit einer Kompressionsbinde. Die Kompressionsbinde dient dabei als Widerlager für die sogenannte Venen-Muskel-Pumpe. Das Wundversorgungsprodukt wird während der Muskelkontraktion zwischen Körperoberfläche und Kompressionsbinde zusammengedrückt. Bei der nachfolgenden Muskelentspannung kann sich das Wundversorgungsprodukt wieder ausdehnen. Die Muskelanspannung entsteht unwillkürlich beim Gehen. Falls ein Patient bettlägerig ist, können alternativ auch im liegenden Zustand geeignete Übungen durchgeführt werden - beispielsweise unter Anleitung eines Physiotherapeuten. Auch in den hier genannten Fällen tritt ein vorteilhafter verstärkter Spüleffekt auf.

Im Sinne dieses Aspekts (verstärkter Spüleffekt) kann der Ausdruck wiederholte reversible Verformung oder wiederholte Druckkompression bedeuten, dass das Wundversorgungsprodukt in der Lage ist, die Wunde verstärkt zu spülen, wenn es mindestens drei Mal verformt oder komprimiert wird und dabei jedes Mal ohne zusätzliche Unterstützung im Wesentlichen in seine Ausgangsform zurückkehrt, wobei eine verstärkte Spülung als eine solche zu verstehen ist, bei der mehr Volumen an wässriger Salzlösung abgegeben wird (z.B. an eine Wunde oder eine Messvorrichtung) im Vergleich zu einem rein passiven Stoffaustausch (ohne Verformung oder Kompression) entlang des Konzentrationsgradienten. Das Wundversorgungsprodukt kann hierbei im Ausgangszustand z.B. zu 80 % der maximalen Absorptionskapazität mit der wässrigen Salzlösung gesättigt sein.

Im Kontext der Erfindung ist das Wundversorgungsprodukt in der Lage während der Wundbehandlung einen muskulär angetriebenen Pumpeffekt zur Spülung der zu behandelnden Wunde mit der in der Quellschicht enthaltenen wässrigen Salzlösung zu erzeugen, und zwar insbesondere beim Gehen, wenn das Produkt an der Fußsohle fixiert ist.

Der auf diese Weise intensivierte Spüleffekt ermöglicht eine äußerst schonende Reinigung der Wunde von Nekrosen und Fibrinbelägen, wodurch in der Regel eine für den Patienten belastende chirurgische invasive Reinigung mittels Skalpell, scharfem Löffel etc. vermieden werden kann.

Bevorzugt stellt der verstärkte Spüleffekt gleichzeitig einen verstärkten Saug-/Spüleffekt dar, der gleichzeitig mit einer verstärkten Aufnahme von aus der Wunde aufgesaugten Stoffen einhergeht. Z.B. werden aus der Wunde ausgespülte Mikroorganismen in der Quellschicht zurückgehalten und beim nächsten Verbandswechsel entfernt.

Weiterhin kann vorgesehen sein, dass nach dem Zuschneiden des erfindungsgemäßen Wundversorgungsproduktes die strukturelle Integrität des verbliebenen, für die Wundversorgung vorgesehenen Bereiches erhalten bleibt, so dass aufgrund der Filzstruktur der Quellschicht keine faser- oder partikelförmigen Bestandteile des zugeschnittenen Wundversorgungsprodukts, insbesondere keine superabsorbierenden Fasern, freigesetzt werden. Ein solches Wundversorgungsprodukt ist somit fusselfrei, und zwar auch nach einem Zuschneiden unmittelbar vor der Anwendung.

Bei dem erfindungsgemäßen Wundversorgungsprodukt kann vorgesehen sein, dass der pH-Wert der wässrigen Salzlösung von weniger als 7,0 durch eine in den superabsorbierenden Fasern enthaltene Säuregruppe, bevorzugt durch Acrylsäure, vermittelt wird. Diese Säuregruppe kann Teil des Polymers sein, welches in den SAF enthalten ist oder aus welchem die SAF bestehen. Bei dem Polymer kann es sich um Polyacrylsäure handeln. Der Vorteil ist, dass auf weitere Bestandteile zur Ansäuerung verzichtet werden kann und ein für die allermeisten Wunden heilungsförderlicher pH-Wert im leicht sauren Bereich eingestellt werden kann. Bevorzugt liegt der durch die besagte Säuregruppe vermittelte pH-Wert der wässrigen Salzlösung im Bereich von 4,5 bis 6,9. Besonders bevorzugt liegt der pH-Wert im Bereich von 5,0 bis 6,5.

Ferner ist bevorzugt vorgesehen, dass die erste Schicht, die Quellschicht und die zweite Schicht bei Flüssigkeitsaufnahme eine im Wesentlichen identische laterale Größenzunahme aufweisen. Im Wesentlichen identische laterale Größenzunahme meint, dass sich die flächige Ausdehnung dieser Schichten in demselben Maße vergrößern, wenn sie Feuchtigkeit aufnehmen, wobei geringe Flächenunterschiede von bis zu 5 %, bevorzugt von bis zu 3 % zwischen den jeweils aneinandergrenzenden Schichten möglich und miterfasst sind. Die Ausdehnung kann gemäß dem Standard AATCC TM 135 bestimmt werden. Bezüglich einer möglichen Schrumpfung nach Flüssigkeitsabgabe gilt dies analog in Bezug auf die Verkleinerung der Flächen der Schichten.

Der Vorteil einer im Wesentlichen gleichen Flächenveränderung der drei Schichten bei Kontakt mit Flüssigkeit ist, dass es in Folge der Feuchtigkeitsaufnahme nicht zu einer Verformung (z.B. Krümmung oder Ausstülpung) des Wundversorgungsproduktes kommt und die planare Form erhalten bleibt.

In diesem Kontext können erste Schicht, Quellschicht und zweite Schicht auch denselben oder im Wesentlichen denselben thermischen lateralen Ausdehnungskoeffizienten haben, wobei sich dieser auf die Flächenausdehnung bezieht. Unterschiede von bis zu 5 %, bevorzugt von bis zu 3 % zwischen den jeweils aneinandergrenzenden Schichten sind als Toleranzen miterfasst. Ein identischer oder im Wesentlichen identischer thermischer lateraler Ausdehnungskoeffizient bietet einen Vorteil bei einer Hitze-Sterilisation des Wundversorgungsproduktes. Andernfalls kann es zu unerwünschten Verformungen des Produktes während der Sterilisation kommen und die Sterilisation muss mit anderen (in der Regel aufwändigeren oder teureren) Verfahren durchgeführt werden, beispielsweise Bestrahlung oder Begasung mit Ethylenoxid.

Eine identische oder im Wesentlichen identische Ausdehnung bzw. thermische Ausdehnung kann dadurch erreicht werden, dass mehreren Schichten Materialien zum Einsatz kommen, die dasselbe oder ein sehr ähnliches Ausdehnungsverhalten zeigen. So zeigen synthetische Fasern sowohl untereinander als auch im Vergleich zu Baumwolle ein sehr ähnliches Ausdehnungs- und Schrumpfungsverhalten.

Erfindungsgemäß ist vorgesehen, dass die Quellschicht des Wundversorgungsproduktes neben SAF mindestens eine andere Faserart bzw. mindestens eine Zusatzfaser umfasst. Hierbei handelt es sich vorteilhaft um eine zellulosehaltige Faser wie zum Beispiel Lyocell oder um eine Polyolefin-basierte Faser, die beispielsweise PE enthält.

Lyocell-Fasern haben den Vorteil eines hervorragenden Feuchtigkeitsspeicherungsvermögens, welches die bereits hohe Kapazität von Baumwolle noch übertrifft. Somit unterstützen Lyocell-Fasern die SAF in synergistischer Weise, verleihen der Quellschicht die nötige strukturelle Belastbarkeit und halten die SAF innerhalb der Quellschicht an Ort und Stelle.

Weiterhin ist es im Hinblick auf das erfindungsgemäße Wundversorgungsprodukt möglich, dass der Vliesstoff der ersten Schicht und / oder der zweiten Schicht Viskose und Polyester enthält oder aus diesen Polymeren besteht. Dabei kann der Polyesteranteil im Vliesstoff beispielsweise 30 bis 50 Massenprozent betragen. Durch die Beimischung des hydrophoben Polyesters entsteht sowohl ausgehend von der ersten Schicht als auch der zweiten Schicht ein hygroskopischer Gradient, der Flüssigkeit durch die beiden zuletzt genannten Schichten in Richtung der Quellschicht durchleitet. Gleichzeitig kann die wässrige Salzlösung von der Quellschicht kommend die erste sowie zweite Schicht passieren und dann in die Wunde gelangen, ohne in der ersten oder zweiten Schicht zurückgehalten zu werden.

Ferner ist es möglich, dass die erste Schicht und die zweite Schicht aus einem identischen Material oder einer identischen Materialmischung bestehen und / oder dasselbe Flächengewicht haben. Dabei kann es sich um solche Materialien oder Materialgemische handeln, die im letzten Absatz aufgeführt sind. Die Verwendung identischer Materialien für beide Schichten garantiert, dass beide Schichten dieselben Eigenschaften haben. Dies gilt umso mehr bei demselben Flächengewicht. Auf diese Weise werden Bedienungsfehler durch den Anwender vermieden, bei welchen die falsche bzw. nicht die optimale Seite auf die Wunde gelegt wird. Daneben bietet ein solcher Aufbau beim Tamponieren einer Kavität den Vorteil, dass sämtliche Wundwände (unabhängig von ihrer räumlichen Lage) mit identischem Material kontaktiert und behandelt werden, was zu einem konstanten Behandlungseffekt bei jedem Einsatz führt.

Generell ist möglich, dass der Vliesstoff der ersten Schicht und / oder der zweiten Schicht im trockenen Zustand ein Flächengewicht von 15 bis 50 g/m2, beispielsweise 18 bis 45 g/m2, hat. Das Flächengewicht kann anhand der Norm DIN EN 12127 bestimmt werden. Es hat sich gezeigt, dass derart beschaffene Schichten eine sehr gute strukturelle Belastbarkeit mit einer hohen Durchlässigkeit für Flüssigkeiten kombinieren. Die Schichten bleiben auch nach einem möglichen Zuschneiden belastbar, behindern jedoch nicht den gewünschten Saug-/Spüleffekt des Wundversorgungsproduktes.

Das erfindungsgemäße Wundversorgungsprodukt kann unterschiedliche äußere Formen und Dimensionen haben. Für die Behandlung einer (äußeren) Wunde wird eine Form empfohlen, die beidseitig (also sowohl proximal als auch distal) flach ist. Für die Behandlung von Ulcera an der Fußsohle ist darüber hinaus vorteilhaft, das Wundversorgungsprodukt mit einer geringen Höhe auszugestalten. Eine solche Wundversorgung stört den Patienten kaum oder gar nicht in seinem üblichen Tagesablauf, da der Patient das Produkt unter Socken und in Schuhen tragen und sogar damit gehen kann. Eine solche geringe Höhe beträgt 1,5 mm bis 6 mm. Sollte das Wundversorgungsprodukt lediglich den Mindestaufbau aus erster Schicht, Quellschicht, zweiter Schicht und optionalen Klebeschichten aufweisen, kann die Höhe noch vorteilhaftere 1,7 mm bis 3 mm, bevorzugt 1,7 mm bis 2,5 mm und besonders bevorzugt 1,8 mm bis 2,2 mm betragen. Die angegebenen Bereichsangaben für die Höhe beziehen sich auf den in der vorliegenden Anmeldung beschrieben trockenen Verbund (also ohne wässriger Salzlösung) aus erster Schicht, Quellschicht und zweiter Schicht, und gleichfalls ohne optionale Release-Liner oder sonstiges Verpackungsmaterial. Die Höhe des mit der wässrigen Salzlösung behandelten Produktes kann (in Abhängigkeit von dem beaufschlagten Volumen) in einer etwa 10 %-igen Steigerung der Höhe resultieren. Folglich hätte das Wundversorgungsprodukt im feuchten Zustand beispielsweise eine Höhe von 1,7 mm bis 6,6 mm, besser 1,9 mm bis 3,3 mm, noch besser 1,9 mm bis 2,8 mm und am besten 2 mm bis 2,4 mm.

Beispiele für weitere mögliche Formen sind kissenförmig und zylinderförmig. Die kissenförmige Variante sorgt für einen besonders guten Polsterungseffekt. Dies wird beispielsweise im Falle von am Knöchel befindlichen Wunden von Patienten als besonders angenehm empfunden. Die zylinderförmige Variante ist besonders vorteilhaft bei tunnelförmigen Wunden und Cavitäten, wobei ein eventuell abstehendes (aus der Wunde herausragendes) Ende einfach abgeschnitten werden kann. Auf diese Wiese wird ein vorschneller oberflächlicher Wundverschluss mit darunterliegender Einkapselung und nachfolgender Abszessbildung verhindert.

Was die Form angeht, so kann das Wundversorgungsprodukt in der Aufsicht rund, rechteckig, quadratisch, oval oder rautenförmig sein. Die Fläche kann beispielsweise 50 cm2 bis 500 cm2 betragen, wobei größere Flächen den Vorteil bieten auf die jeweilige Wundgröße zugeschnitten werden zu können.

Die Quellschicht des Wundversorgungsproduktes enthält vorzugsweise die wässrige Salzlösung in einer Menge, die die Absorptionskapazität der Quellschicht nicht ausschöpft. Das heißt, dass die Quellschicht vorzugsweise nicht gesättigt bzw. ungesättigt ist. Auf diese Weise kann die Quellschicht bzw. das Wundversorgungsprodukt weitere Flüssigkeit wie z.B. Wundexsudat oder Blut aufnehmen. So ist es möglich, dass die Quellschicht oder alternativ das ganze Wundversorgungsprodukt zu maximal 50%, maximal 60%, maximal 70%, maximal 80%, maximal 90% oder maximal 95% mit der wässrigen Salzlösung gesättigt ist.

Welcher von diesen Werten am besten geeignet ist, hängt von der zu versorgenden Wundart ab. Je stärker eine Wunde mit Belegen (z.B. Fibrinbelegen), nekrotischem Gewebe oder Biofilm bedeckt ist, desto mehr wird diese Wunde von einer intensiven Spülung profitieren, so dass sich in einem solchen Fall hohe Sättigungswerte von 80% oder mehr empfehlen. Hingegen können stark exsudierende, nicht infizierte Wunden mit solchen Varianten des Produktes behandelt werden, die eine geringere Sättigung im Bereich vom 50% bis 70% aufweisen.

Die angegeben Sättigungswerte sollen nachfolgend an einem Beispiel erklärt werden: Wenn die Quellschicht maximal 100 ml einer wässrigen Salzlösung (osmotischer Wert im isotonischen Bereich, also 9 g NaCl auf einen Liter H2O) absorbieren kann, so ist die Quellschicht nach Aufnahme von 50 ml zu 50% gesättigt.

Weiterhin können die angegeben Sättigungswerte sich auch auf ein Wundversorgungsprodukt mit den drei Hauptschichten (erster Schicht, Quellschicht, zweiter Schicht und optionale dazwischenliegende Klebeschichten) beziehen oder auf das gesamte Wundversorgungsprodukt, welches je nach Bedarf auch über zusätzliche Schichten verfügen kann.

Ferner kann bestimmt sein, dass das Wundversorgungsprodukt nicht geeignet oder kompatibel zur Unterdrucktherapie ist, da es beispielsweise keinen Port hat, über welchen ein Unterdruck an das Produkt bzw. eine Wunde unterhalb des Produktes angelegt werden kann.

Im Rahmen der vorliegenden Erfindung kann vorgesehen sein, dass das Wundversorgungsprodukt keine superabsorbierenden Partikel enthält. Bevorzugt sind überhaupt keine Partikel enthalten, die nach dem Zuschneiden des Produktes herausfallen könnten. Ausgenommen hiervon sind Klebestoffpartikel, die während der Herstellung des Produktes eingesetzt werden können, da sich diese mit den übrigen Materialien verbinden und dem gewünschten Effekt der Zuschneidbarkeit nicht entgegenstehen. Auch Wirkstoffpartikel, die sich in der wässrigen Salzlösung auflösen, sind nicht vom Erfindungskonzept ausgenommen.

Bei dem in der wässrigen Salzlösung vorhanden Salz kann es sich um NaCl handeln. Bevorzugt ist die wässrige Salzlösung isotonisch. Eine solche isotonische Salzlösung hat im Wesentlichen denselben osmotischen Druck wie menschliches Blut. Geringe Abweichungen des Druckwerts von 1% in Pascal nach oben bzw. unten sind dabei tolerierbar und fallen ebenfalls unter den Begriff der isotonischen Lösung.

Die wässrige Salzlösung kann über NaCl hinaus auch andere Salze wie KCL oder Salzkombinationen wie beispielsweise KCI und NaCl enthalten. Eine bevorzugte Variante der wässrigen Salzlösung ist die sogenannte Ringerlösung, welche neben NaCl und KCI auch CaCl enthält und isotonisch ist.

Eine isotonische Lösung bietet den Vorteil, dass die ungeschützten Zellen im offenen Bereich der zu behandelnden Wunde keinem osmotischen Stress ausgesetzt werden. Vielmehr wirkt sich die Zufuhr der in der Salzlösung enthaltenen Mineralstoffe positiv auf den Zellmetabolismus aus. So sind menschliche Zellen in der Lage über membranständige Proteine wie beispielsweise die Aquaporine Mineralien aus der Umgebung aufzunehmen.

Die in der Quellschicht eingelagerte wässrige Salzlösung kann von der Quellschicht in die übrigen Schichten des Wundversorgungsproduktes übertreten. Insbesondere kann die wässrige Salzlösung in der ersten Schicht und der zweiten Schicht vorhanden sein. Dies beeinträchtigt die Funktionsweise des Produktes nicht. Ein Durchtritt der Lösung durch die erste und / oder zweite Schicht spätestens nach Applikation auf oder in der Wunde ist sogar erwünscht. Bei der Bestimmung der zur Benetzung der Quellschicht notwendigen Lösungsmenge kann die Aufnahmekapazität der übrigen Schichten berücksichtigt werden.

Im Folgenden soll die Erfindung anhand von exemplarischen Zeichnungen näher erläutert werden, wobei die dargestellten Varianten je nach Einsatzzweck mithilfe der in diesem Dokument enthaltenen technischen Informationen abgewandelt werden können.

Figur 1 zeigt eine Ausführungsform des erfindungsgemäßen Wundversorgungsproduktes, welche hervorragend zur Anwendung als Tamponade (d.h. zum Austamponieren von Cavitäten und tiefen Wunden) geeignet ist, im Querschnitt.

Figur 2 zeigt das Produkt von Figur 1 in zugeschnittener Form in der Aufsicht.

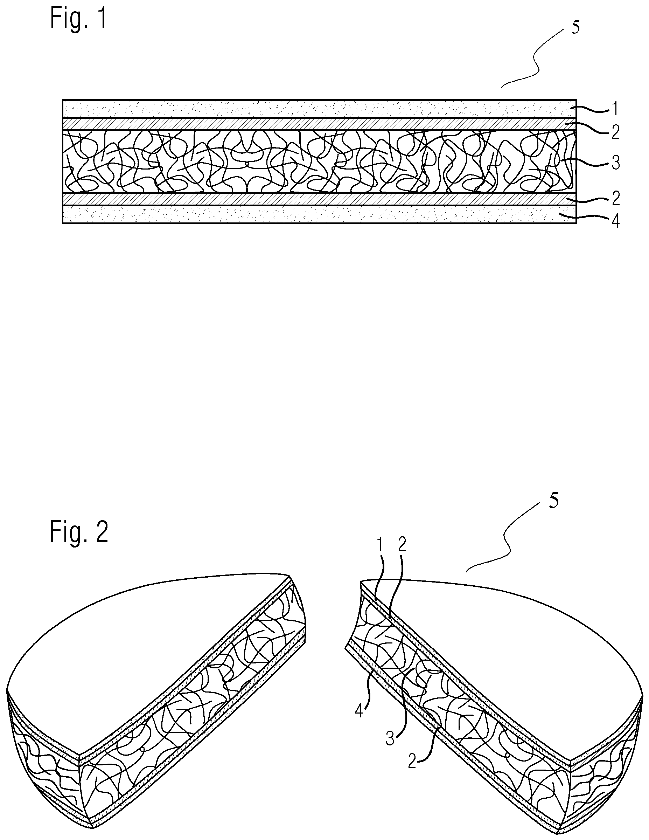

In den Figuren 1 und 2 ist ein erfindungsgemäßes Wundversorgungsprodukt (5) abgebildet, umfassend eine oben zweite flüssigkeitsdurchlässige Schicht (1) aus Vliesstoff, die mittels einer Klebestoffschicht (2) an der Quellschicht (3) befestigt ist. Innerhalb der Quellschicht befindet sich ein Faserverbund, der miteinander vernadelte superabsorbierende Fasern und Zusatzfasern enthält, wobei die Zusatzfasern keine superabsorbierenden Fasern sind. Die Quellschicht (3) ist mit einer wässrigen Salzlösung behandelt (nicht dargestellt). Unterhalb der Quellschicht (3) befindet sich die erste flüssigkeitsdurchlässige Schicht (4). Quellschicht (3) und erste Schicht (4) sind mittels einer Klebestoffschicht (2) zusammengefügt. In der Darstellung von Figur 2 ist das Wundversorgungsprodukt (5) mittig in zwei Hälften (Segmente) zerschnitten. Die strukturelle Einheit sowie Funktionalität einer jeden Hälfte bleiben gewahrt.

### Beispiele

Nachfolgend wird die Erfindung anhand von Ausführungsbeispielen näher erläutert:

### Beispiel 1: Herstellung eines Wundversorgungsproduktes zur Anwendung als Tamponade

Zunächst wurde eine Quellschicht (4) als Meterware bereitgestellt. An der Unterseite der Quellschicht (4) wurde eine erste flüssigkeitsdurchlässige Schicht (4) in Form von Vliesstoff aus PP angebracht. An der Oberseite der Quellschicht wurde eine zweite flüssigkeitsdurchlässige Schicht (1) angebracht, welche ebenfalls aus einem PP-Vliesstoff bestand. Zur Befestigung der Schichten aneinander wurden Klebepartikel in Form von biologisch abbaubarem Polyester zwischen den Schichten aufgetragen. Die Klebepartikel wurden in einem nachfolgendem thermischen Verfestigungsprozess aneinander befestigt.

Dies geschah mittels thermischer Behandlung in einem Trockenofen bei einer Temperatur von mehr als 120°C mit anschließender Walzenkompression. Das derart erzeugte und aus drei Schichten bestehende Laminat hatte ein Flächengewicht von 260 g/m2 bei einer Dicke von 2,3 mm. Im nächsten Schritt wurde das Laminat auf eine Rolle aufgewickelt und von dieser Rolle ausgehend in eine sogenannte Convertingmaschine eingespeist, wo quadratische Ausschnitte mit einer Fläche von 10 cm x10 cm aus dem Laminat herausgetrennt wurden.

Anschließend wurden die Produkte mittels einer Verpackungsmaschine prozessiert. In der Verpackungsmaschine wurden die bis dahin trockenen Produkte auf einer flüssigkeitsdichten Verpackungsfolie ausgebreitet. Es wurde wässrige Salzlösung hinzugefügt, und zwar in einer Menge, die die maximale Absorptionskapazität der Produkte nicht ausschöpfte (ca. 31 ml). Es folgte die Versiegelung mit einer oberen Verpackungsfolie (flüssigkeitsdicht), so dass die Wundversorgungsprodukte (5) zwischen unterer und oberer Folie verschlossen waren. Danach wurde der so entstandene Beutel mit den darin enthaltenen Produkten bei 120°C dampfsterilisiert. Anschließend wurden die nun feuchten Wundversorgungsprodukte (5) im Beutel, der gleichzeitig als Teil des Verpackungsmaterials fungierte, steril vorrätig gehalten. Die so erhaltenen sterilen Produkte (5) können nach der Entnahme aus dem Beutel für das Tamponieren von Wunden (z.B. Kavitäten) eingesetzt werden.

### Beispiel 2: Zuschneiden der Wundversorgungsprodukte

Das Wundversorgungsprodukt (5) gemäß Beispiel 1 wurde jeweils ca. in der Mitte (d.h. etwa 5 cm entfernt vom linken bzw. rechten Rand) mit einer handelsüblichen Schere in zwei Hälften geschnitten. Der Schnittvorgang konnte mühelos und ohne besonderen Kraftaufwand durchgeführt werden. Die Schnittkanten wurden visuell beurteilt. Es konnten keinerlei Ausfransungen oder lose Fasern festgestellt werden. Die Schnittflächen waren eben, glatt und geradlinig.

Darüber hinaus bildeten die Schichten der Zuschnitte weiterhin einen stabilen Verbund und zeigten keinerlei Tendenz zu Trennung, Zerfall oder Delaminierung. Somit war jeder einzelne Zuschnitt geeignet, um in der Wundversorgung eingesetzt zu werden.

### Beispiel 3: Messung der maximalen Druckbelastung

Es wurde ein etwa zwei Wochen altes feuchtes Wundversorgungsprodukt (5) gemäß Beispiel 1 - jedoch kreisförmig - bereitgestellt und in einen handelsüblichen transparenten Plastik-ZIP-Beutel gelegt. Der Beutel wurde verschlossen und für den weiteren Versuchsablauf in ein Kalibriergerät der Firma Sensomative eingelegt. Das Wundversorgungsprodukt (5) war kreisförmig (in der Aufsicht) mit einem Durchmesser von 4,5 cm. Anschließend wurden Belastungszyklen gefahren, bei denen jeweils innerhalb von 1 Sekunde eine Gewichtskraft von 188.5 N (entsprechend etwa 19 kg) auf die gesamte Fläche des Produkts aufgebaut wurde. Die Gewichtskraft wurde für die Dauer von einer Sekunde aufrechterhalten und anschließend innerhalb von 2 Sekunden auf 0 N reduziert. Die Anzahl der Belastungszyklen betrug zehn. Der Vorgang fand bei einer Temperatur zwischen 22 °C und 23 °C statt. Die Übertragung der Gewichtskraft fand mithilfe einer im Kalibriergerät verbauten aufblasbaren Hülle aus synthetischem, luftdichten Material statt. Die Hülle wurde während der Belastungszyklen pneumatisch befüllt.

Nach Beendigung des Belastungstests wurde das Produkt (5) aus der Anlage entfernt. Sowohl das Produkt (5) als auch der Beutel wurden visuell inspiziert. Es konnte kein Materialbruch festgestellt werden. Sämtliche Schichten (1, 3, 4) des Produktes (5) waren strukturell intakt. Nach der Entnahme des Produktes (5) aus dem Beutel waren weder am Produkt (5) gerissene Fasern oder ausgetretenes Gel sichtbar noch konnten abgetrennte oder ausgetretene Materialien im Beutelinneren festgestellt werden. Lediglich eine geringe Menge der wässrigen Salzlösung ist im Beutel zurückgeblieben.

## Patentansprüche

1. Zuschneidbares Wundversorgungsprodukt (9), enthaltend
a) eine erste flüssigkeitsdurchlässige Schicht (4) mit einem Vliesstoff,
b) eine zweite flüssigkeitsdurchlässige Schicht (1) mit einem Vliesstoff,
c) eine zwischen der ersten Schicht (4) und der zweiten Schicht (1) gelegene Quellschicht (3) in Form eines Vliesstoffes mit superabsorbierenden, polymerhaltigen Fasern, wobei die superabsorbierenden Fasern mit mindestens einer anderen Faserart in einem Filz vorliegen,
d) eine wässrige Salzlösung, die in der Quellschicht (3) eingelagert ist, im eingelagerten Zustand einen pH < 7,0 hat und die während einer Wundbehandlung an eine Wunde abgegeben werden kann,
wobei die erste Schicht (4), die Quellschicht (3) und die zweite Schicht (1) einen identischen Zuschnitt haben und ohne Überstand vollflächig aneinanderliegen, und wobei das Wundversorgungsprodukt (9) nahtlos ausgestaltet ist.

2. Zuschneidbares Wundversorgungsprodukt (9) nach Anspruch 1, wobei das Wundversorgungsprodukt (9) eine Druckresistenz gegenüber einem Druck von mindestens 8 kg / 100 cm² aufweist, so dass durch Einwirkung eines solches Drucks das Wundversorgungsprodukt (9) lediglich elastisch verformt wird.

3. Zuschneidbares Wundversorgungsprodukt (9) nach Anspruch 1 oder 2, wobei die andere Faserart ausgewählt ist aus der Gruppe bestehend aus: zellulosehaltige Faser, polyesterhaltige Faser, bevorzugt Polyethylenterephthalat und / oder Polyamid und wobei die andere Faserart mit den superabsorbierenden, polymerhaltigen Fasern vernadelt ist, so dass sie in einem Nadelfilz vorliegt.

4. Zuschneidbares Wundversorgungsprodukt (9) nach einem der vorhergehenden Ansprüche, wobei der Vliesstoff der ersten Schicht (4) und / oder der zweiten Schicht (1) thermisch verfestigte Fasern enthält.

5. Zuschneidbares Wundversorgungsprodukt (9) nach Anspruch 4, wobei die thermisch verfestigten Fasern ein oder mehrere Polymere ausgewählt aus Polypropylen, PolyVinyl-Chlorid, Polyethylen, Polyethylenterephthalat, Polycarbonat, Polyamid, Polyurethan, Polystyrol, sowie Mischungen daraus, enthalten oder daraus bestehen.

6. Zuschneidbares Wundversorgungsprodukt (9) nach einem der vorhergehenden Ansprüche, wobei die superabsorbierenden Fasern in der Quellschicht (3) ein Polymer, bevorzugt ein acrylathaltiges Polymer oder ein Acrylat-Co-Polymer, enthalten, welches quervernetzbar ist, wobei die Quervernetzbarkeit bevorzugt auf der Bildung von Ester-Verbindungen beruht.

7. Zuschneidbares Wundversorgungsprodukt (9) nach einem der vorhergehenden Ansprüche, wobei die erste Schicht (4), die Quellschicht (3) und die zweite Schicht (1) als Laminat vorliegen.

8. Zuschneidbares Wundversorgungsprodukt (9) nach einem der vorhergehenden Ansprüche, wobei die erste Schicht (4), die Quellschicht (3) und die zweite Schicht (1) flächig unlösbar miteinander verbunden sind, wobei die Schichten vorzugsweise durch einen Schmelzkleber zusammengefügt sind, und wobei der Schmelzkleber vorzugsweise ein Polyolefin oder Copolymer enthält oder daraus besteht.

9. Zuschneidbares Wundversorgungsprodukt (9) nach einem der vorhergehenden Ansprüche, wobei beim Zuschneiden die strukturelle Integrität des Wundversorgungsproduktes (9) erhalten bleibt, so dass durch die Filzstruktur der Quellschicht keine faser- oder partikelförmigen Bestandteile des Wundversorgungsproduktes (9), insbesondere keine superabsorbierenden Fasern, freigesetzt werden.

10. Zuschneidbares Wundversorgungsprodukt (9) nach einem der vorhergehenden Ansprüche, wobei der Vliesstoff der ersten Schicht (4) und / oder der zweiten Schicht (1) im trockenen Zustand ein Flächengewicht von 15 g/m² bis 50 g/m² hat.

11. Zuschneidbares Wundversorgungsprodukt (9) nach einem der vorhergehenden Ansprüche, wobei das Wundversorgungsprodukt (9) eine Höhe von 1,7 mm bis 6,6 mm hat.

12. Zuschneidbares Wundversorgungsprodukt (9) nach einem der vorhergehenden Ansprüche, wobei die Quellschicht zu maximal 95 % mit der wässrigen Salzlösung gesättigt ist.

13. Zuschneidbares Wundversorgungsprodukt (9) nach einem der vorhergehenden Ansprüche, wobei es sich bei der wässrigen Salzlösung um NaCl, KCI und CaCl enthält.

14. Zuschneidbares Wundversorgungsprodukt (9) nach einem der vorhergehenden Ansprüche, wobei der pH-Wert der in der Quellschicht eingelagerten wässrigen Salzlösung im Bereich von 4,5 bis 6,9 liegt.

15. Zuschneidbares Wundversorgungsprodukt (9) nach einem der vorhergehenden Ansprüche, wobei die erste Schicht (4) und die zweite Schicht (1) aus einem identischen Material oder einer identischen Materialmischung bestehen.

## Claims

1. Trimmable wound care product (9) containing
a) a first liquid-permeable layer (4) comprising a nonwoven,
b) a second liquid-permeable layer (1) comprising a nonwoven,
c) a swelling layer (3) between the first layer (4) and the second layer (1) and in the form of a nonwoven comprising superabsorbent, polymer-containing fibres, the superabsorbent fibres being present in a felt with at least one other fibre type,
d) an aqueous saline solution which is stored in the swelling layer (3), which has a pH < 7.0 during storage and which can be delivered to a wound during a wound treatment,
wherein the first layer (4), the swelling layer (3) and the second layer (1) are identically trimmed and lie against each other full face-to-full face without any overhang, and wherein the wound care product (9) is seamless.

2. Trimmable wound care product (9) according to Claim 1, wherein the wound care product (9) has a pressure resistance to a pressure of at least 8 kg / 100 cm², such that the action of such a pressure merely causes elastic deformation of the wound care product (9).

3. Trimmable wound care product (9) according to Claim 1 or 2, wherein the other fibre type is selected from the group consisting of: cellulose-containing fibre, polyester-containing fibre, preferably polyethylene terephthalate, and/or polyamide and wherein the other fibre type is needled with the superabsorbent, polymer-containing fibres, such that it is present in a needle felt.

4. Trimmable wound care product (9) according to any of the preceding claims, wherein the nonwoven of the first layer (4) and/or of the second layer (1) contains thermally consolidated fibres.

5. Trimmable wound care product (9) according to Claim 4, wherein the thermally consolidated fibres contain or consist of one or more polymers selected from polypropylene, polyvinyl chloride, polyethylene, polyethylene terephthalate, polycarbonate, polyamide, polyurethane, polystyrene, and mixtures thereof.

6. Trimmable wound care product (9) according to any of the preceding claims, wherein the superabsorbent fibres in the swelling layer (3) contain a polymer, preferably an acrylate-containing polymer or an acrylate copolymer, which is crosslinkable, the crosslinkability being preferably based on the formation of ester bonds.

7. Trimmable wound care product (9) according to any of the preceding claims, wherein the first layer (4), the swelling layer (3) and the second layer (1) are in the form of a laminate.

8. Trimmable wound care product (9) according to any of the preceding claims, wherein the first layer (4), the swelling layer (3) and the second layer (1) are inseparably bonded to each other face-to-face, the layers being preferably joined together by means of a hot-melt adhesive and the hot-melt adhesive preferably containing or consisting of a polyolefin or copolymer.

9. Trimmable wound care product (9) according to any of the preceding claims, wherein the structural integrity of the wound care product (9) is maintained on trimming, such that the felt structure of the swelling layer does not release fibrous or particulate constituents of the wound care product (9), in particular superabsorbent fibres.

10. Trimmable wound care product (9) according to any of the preceding claims, wherein the nonwoven of the first layer (4) and/or of the second layer (1) in the dry state has a basis weight of 15 g/m² to 50 g/m².

11. Trimmable wound care product (9) according to any of the preceding claims, wherein the wound care product (9) has a height of 1.7 mm to 6.6 mm.

12. Trimmable wound care product (9) according to any of the preceding claims, wherein the swelling layer is saturated with the aqueous saline solution to an extent of not more than 95%.

13. Trimmable wound care product (9) according to any of the preceding claims, wherein the aqueous saline solution contains NaCl, KCl and CaCl.

14. Trimmable wound care product (9) according to any of the preceding claims, wherein the pH of the aqueous saline solution stored in the swelling layer is in the range from 4.5 to 6.9.

15. Trimmable wound care product (9) according to any of the preceding claims, wherein the first layer (4) and the second layer (1) consist of an identical material or an identical material blend.

## Revendications

1. Produit de soin de plaies (9) pouvant être découpé à la bonne taille, contenant
a) une première couche perméable aux liquides (4) avec un non-tissé,
b) une deuxième couche perméable aux fluides (1) avec un non-tissé,
c) une couche de gonflement (3) placée entre la première couche (4) et la deuxième couche (1) sous la forme d'un non-tissé avec des fibres superabsorbantes contenant des polymères, les fibres superabsorbantes avec au moins un autre type de fibres étant présentes dans un feutre,
d) une solution saline aqueuse, qui est stockée dans la couche de gonflement (3), présente un pH < 7,0 lorsqu'elle est stockée et qui peut être administrée à une plaie pendant le traitement de la plaie ;
la première couche (4), la couche de gonflement (3) et la deuxième couche (1) présentant une découpe identique et reposant totalement les uns sur les autres sans débord, et le produit de soin de plaies (9) étant conçu sans couture.

2. Produit de soin de plaies (9) pouvant être découpé à la bonne taille selon la revendication 1, le produit de soin de plaies (9) présentant une résistance à la pression par rapport à une pression d'au moins 8 kg / 100 cm², de telle sorte que le produit de soin de plaies (9) est déformé seulement élastiquement par l'action d'une telle pression.

3. Produit de soin de plaies (9) selon la revendication 1 ou 2, l'autre type de fibres étant choisi dans le groupe constitué par : des fibres contenant de la cellulose, des fibres contenant du polyester, de manière préférée du polytéréphtalate d'éthylène et/ou du polyamide, et l'autre type de fibres étant aiguilleté avec les fibres superabsorbantes contenant un polymère, de telle sorte qu'il est présent dans un feutre aiguilleté.

4. Produit de soin de plaies (9) pouvant être découpé à la bonne taille selon l'une des revendications précédentes, le non-tissé de la première couche (4) et/ou de la deuxième couche (1) contenant des fibres thermiquement solidifiées.

5. Produit de soin de plaies (9) découpé à la bonne taille selon la revendication 4, les fibres solidifiées thermiquement contenant un ou plusieurs polymères choisis parmi le polypropylène, le polychlorure de vinyle, le polyéthylène, le polytéréphtalate d'éthylène, le polycarbonate, le polyamide, le polyuréthane, le polystyrène ainsi que des mélanges de ceux-ci ou en étant constituées.

6. Produit de soin de plaies (9) pouvant être découpé à la bonne taille selon l'une des revendications précédentes, les fibres superabsorbantes dans la couche de gonflement (3) contenant un polymère, de manière préférée un polymère contenant un acrylate ou un copolymère acrylate, qui peut être réticulé, l'aptitude à la réticulation reposant de manière préférée sur la formation de composés esters.

7. Produit de soin de plaies (9) pouvant être découpé à la bonne taille selon l'une des revendications précédentes, la première couche (4), la couche de gonflement (3) et la deuxième couche (1) étant présentes sous forme de stratifié.

8. Produit de le soin de plaies (9) selon l'une des revendications précédentes, la première couche (4), la couche de gonflement (3) et la deuxième couche (1) étant reliées les unes aux autres indissolublement à plat, les couches étant de préférence réunies entre elles par un adhésif thermofusible, et l'adhésif thermofusible contenant de préférence une polyoléfine ou un copolymère ou étant constitué de ceux-ci.

9. Produit de soin de plaies (9) pouvant être découpé à la bonne taille selon l'une des revendications précédentes, l'intégrité structurelle du produit de soin de plaies (9) étant préservée lors de la découpe à la bonne taille, de telle sorte qu'aucun composant fibreux ou particulaire du produit de soin de plaies (9), en particulier aucune fibre superabsorbante, n'est libéré par la structure de feutre de la couche de gonflement.

10. Produit de soin de plaies (9) pouvant être découpé à la bonne taille selon l'une des revendications précédentes, le non-tissé de la première couche (4) et/ou de la deuxième couche (1) présentant à l'état sec un poids surfacique de 15 g/m² à 50 g/m².

11. Produit de soin de plaies (9) pouvant être découpé à la bonne taille selon l'une des revendications précédentes, le produit de soin de plaies (9) présentant une hauteur de 1,7 mm à 6,6 mm.

12. Produit de soin de plaies (9) pouvant être découpé à la bonne taille selon l'une des revendications précédentes, la couche de gonflement étant saturée jusqu'à un maximum de 95 % avec la solution saline aqueuse.

13. Produit de soin de plaies (9) pouvant être découpé à la bonne taille selon l'une des revendications précédentes, la solution saline aqueuse contenant NaCl, KCl et CaCl.

14. Produit de soin de plaies (9) selon l'une des revendications précédentes, la valeur de pH de la solution saline aqueuse stockée dans la couche de gonflement se situant dans la plage de 4,5 à 6,9.

15. Produit de soin de plaies (9) selon l'une des revendications précédentes, la première couche (4) et la deuxième couche (1) étant constituées d'un matériau identique ou d'un mélange de matériaux identique.
